# EUROPEAN PATENT SPECIFICATION

(11) **EP 2 813 566 B1**
(45) Date of publication and mention of the grant of the patent: **22.03.2017**
(21) Application number: 13172135.9
(22) Date of filing: 14.06.2013
(51) Int. Cl.: C12N 1/16, A61K 31/415, A61K 36/06, A61K 9/50, A61K 31/352, A61K 31/353, A61K 36/064

(54) **Method for incorporating a compound into live yeast cells**
Verfahren zur Aufnahme einer Verbindung in lebende Hefezellen
Procédé d'incorporation d'un composé dans des cellules de levure vivantes

(43) Date of publication of application: 17.12.2014
(73) Proprietor: Ormige SA, 6000 Charleroi (BE)
(72) Inventor: Doyen, Gérald, 7190 Marche-lez-Écaussinnes (BE)
(74) Representative: Gevers & Orès

(56) References cited:
- SHI G ET AL: "Characterization of yeast cells as a microencapsulation wall material by Fourier-transform infrared spectroscopy", VIBRATIONAL SPECTROSCOPY, ELSEVIER SCIENCE, AMSTERDAM, NL, vol. 53, no. 2, 20 July 2010 (2010-07-20), pages 289-295, XP027061329, ISSN: 0924-2031 [retrieved on 2010-05-18]
- SHI ET AL: "Stabilization and encapsulation of photosensitive resveratrol within yeast cell", INTERNATIONAL JOURNAL OF PHARMACEUTICS, ELSEVIER BV, NL, vol. 349, no. 1-2, 27 December 2007 (2007-12-27), pages 83-93, XP022402470, ISSN: 0378-5173, DOI: 10.1016/J.IJPHARM.2007.07.044
- FEDERICA CIAMPONI ET AL: "Yeast cells as microcapsules. Analytical tools and process variables in the encapsulation of hydrophobes in", APPLIED MICROBIOLOGY AND BIOTECHNOLOGY, SPRINGER, BERLIN, DE, vol. 95, no. 6, 13 May 2012 (2012-05-13), pages 1445-1456, XP035102896, ISSN: 1432-0614, DOI: 10.1007/S00253-012-4127-8

## Description

The present invention relates to a method for incorporating a heat stable compound into a live yeast cell cubltivated under aerobic conditions to produce a biovehicle containing said compound, useful in human or animal food, dietary supplements, immunomodulation or drug delivery applications.

The term probiotic has been firstly defined as "a live microbial feed supplement which beneficially affects the host by improving its intestinal microbial balance" (Fuller, 1989). More recently, Zanello *et al.* (2009) reminded that this definition has been extended to human or animal health. Probiotics were redefined as live micro-organisms that, when administered in adequate amounts, confer a health benefit to the host (report of a Joint FAO/WHO Expert Consultation on Evaluation of Health and Nutritional Properties of Probiotics in Food including Powder Milk with Live Lactic Acid Bacteria, Córdoba, Argentina, 1-4 October 2001).

Diarrhea is a common side effect of antibiotics. It increases treatment costs and length of stay in acute healthcare facilities. One potential strategy to prevent this side effect is the use of probiotic bacteria or yeasts (Hickson, 2011; Johnston BC *et al.,* 2011).

Coming within the scope of improving the intestinal microbial balance during an antibiotic treatment method, a growing number of authors recommend the virtues of a combination therapy between antibiotics and probiotic bacteria or yeasts (International Application WO 2011/044208). Hence, a close association and when feasible a simultaneous administration of an antibiotic and a probiotic in a single formulation can show a better impact on antibiotic-related side effects, than alternate antibiotic then probiotic strain separate ingestion.

Over the past 30 years, yeast has been used as a protective carrier for the delivery of active compounds in human or animal, using a variety of techniques suitable for the compounds being loaded. For instance, inorganic or organic compounds can be inserted (*i.e.,* incorporated) into yeast vehicle by diffusion and/or active transport.

Lipophilic volatile chemicals, as essential oils, can be absorbed into yeasts at high concentrations by a passive diffusion process. The association of temperature and hydrocarbon chain length was used to favour the diffusion across the cell membrane which was the key selective barrier to encapsulation (*i.e.,* incorporation). Bishop *et al.* (1998) have shown that it is unnecessary for the baker's yeast (*Saccharomyces cerevisiae*) cells to be viable for the encapsulation process to occur. Accumulation of material within the yeast cell appeared as small droplets which then appeared to coalesce to convert the majority of the cell volume into a passive reservoir.

Further, the combination of thermal stability and hydration, in particular the octanol-water partition coefficient (log P), facilitates the incorporation within yeast cells then the release of encapsulated flavours (Dardelle, 2007). Indeed, Dardelle (2007) has shown that when empty yeast cells were used as thermo-stable delivery systems for flavour encapsulation, not all the molecules can penetrate inside the yeast. The selection is based on the hydrophobic properties. The efficiency of encapsulation is higher than 50% for flavour compound octanol-water partition coefficient (log P) higher than 2.0.

Other applications for yeast based encapsulated materials have ranged from applying fragrances, biocides and insecticides to textiles (Nelson, 2002) to the use of microencapsulated nicotine in smoking cessation products (International Application WO 00/69440). These applications include insect and weed control in crop and household environments (International Application WO 2005/102045) and targeted drug delivery (Nelson G *et al.,* 2006).

In a recent review, Ciamponi *et al.* (2012) described yeast cells as biocompatible and biodegradable containers for the microencapsulation of a variety of active compounds. They have studied kinetics and mechanistic aspects of the spontaneous internalization (*i.e.,* incorporation) of terpenes (as model hydrophobic compounds) in *S. cerevisiae* and concluded that the encapsulation process is essentially a phenomenon of passive diffusion with negligible relevance of active transport.

There is however a need for improving the methods for incorporating a biologically active compound into a yeast, especially into a live yeast.

The inventors have found that the combination of the culture of a live yeast cell under aerobic conditions in a medium comprising polyphenolic compounds of grape or berry juices (designed as hormetins) and time-extended heat stress favours the incorporation into said yeast cell of heat stable antibacterial agents. More specifically, the thermotolerance coupled with the use of hormetins improve the yeast cell life span and a chimiotolerance to antibacterial compounds. Further, there is no need of growth cycle of the yeast for incorporating the antibiotic compounds. Only one single fermenter flask or tank is also necessary from the start to finish of compound incorporation (no use of incrementally fed stages of fermentation). There is also no need to use lipid substances, organic solvent dehydration or fixation, or plasmolysers to incorporate the compound into the yeast cell.

The inventor has also found that the antibacterial agent *(e.g.,* a macrolide antibiotic) incorporates more easily into the living yeast cells when the hormetin rich medium contains alkaline cereal flour and potato starch and has a pH comprised between 6.0 and 7.0, preferably around or above pH 6.5. This pH around 6.5 is generally obtained when the proportions of red grape juice, cereal alkaline hydrolysate and mashed red or sweet potatoes are equivalent.

A food grade or probiotic yeast strain obtained by this method allows a simultaneous administration to a subject of an antibacterial compound and said yeast strain in a single oral dosage form which shows a better impact on antibacterial compound-related side effects, than antibacterial compound antibiotic then probiotic strain separate ingestion.

Accordingly, the present invention provides a method for incorporating a heat stable biologically active compound for use in human or animal into a live yeast cell cultivated under aerobic conditions, comprising the following steps:
a) cultivating a live yeast cell with a heat stable biologically active compound for use in human or animal, in a hormetin rich medium at a temperature comprised between 20°C and 28°C,
b) increasing the temperature of the culture from the temperature in step a) to a temperature comprised between 39°C and 43°C at a rate of at least 1°C per minute;
c) maintaining the temperature reached in step b) for 60 to 120 minutes;
d) increasing the temperature of the culture from the temperature in step c) to a temperature comprised between 51°C and 53°C at a rate of at least 2°C per minute;
e0) maintaining the temperature reached in step d) for 40 to 80 minutes; and
f) isolating the live yeast cell.

The yeast cell is preferably a non pathogenic yeast strain, more preferably a food grade yeast strain, such as a baker, wine, beer or cheese yeast. The yeast cell can have probiotic properties.

In a preferred embodiment, the yeast cell belongs the family Saccharomycetaceae, such as a *Saccharomyces* strain or a *Kluyveromyces* strain.

Advantageously, the yeast cell is a *Saccharomyces cerevisiae, Saccharomyces bayanus, Saccharomyces boulardii, Saccharomyces uvarum* or a *Kluyveromyces marxianus* strain.

*Saccharomyces cerevisiae* has a generally recognized safe status for human consumption, is easy to cultivate and has a high level of resistance to digestive secretions (Blanquet *et al.,* 2003). *Saccharomyces boulardii* is already commercialized for human use. *K. marxianus* exhibits interesting qualities, such as a broad substrate spectrum, a thermotolerance, high growth rates and less tendency to ferment when exposed to sugar excess (Fonseca *et al.,* 2008).

The yeast strain may also have its own beneficial effect on gut homeostasis (Harms *et al.,* 1987; Martins *et al.,* 2007).

By way of example, the yeast cell is selected from the group consisting of:
- the *Saccharomyces cerevisiae* strain from the vacuum packed Baker's Instant Dry Yeast from Algist Bruggeman, B9000, Ghent,
- *Saccharomyces cerevisiae bayanus* Lalvin QA23, seleccionada na regiao dos vinhos C.R.V.V. U.T.A.D. Portugal, obtained from Lallemand S.A.S. F31702, Blagnac Cedex,
- *Saccharomyces bayanus* Saccardo (syn. *Saccharomyces uvarum* Beijerinck) BCCM-MUCL Industrial fungi and yeasts collection, strain accession number 39513, NCYC 623, CBS 7001,
- *Saccharomyces cerevisiae* (Desmazières) Meyen BCCM-MUCL Industrial fungi and yeasts collection, strain accession number 31345,
- the probiotic *Saccharomyces boulardii,* registered as a medicine in dry powder bags (Ultra-levure 50mg, 100mg or 200mg from Biocodex, F94250, Gentilly, or Enterol 250mg from Impexeco, B7700, Mouscron),
- the lactic yeast *Kluyveromyces marxianus* (E.C. Hansen) Van der Walt var. Marxianus ,also det. Candida Kefyr (Beijerinck) van Uden and Buckley - BCCM-MUCL Industrial fungi and yeasts collection, strain accession number 52258, CCMM-R789.

Advantageously, the yeast cells are inoculated in the hormetin rich medium at 100 to 1000 million cells / ml, preferably at 300 to 800 million cells / ml.

The yeast cells can belong to different strains and can therefore be co-cultured.

Advantageously, two *Saccharomyces* strains can be co-cultured, preferably a S. *cerevisiae* strain and a *S. bayanus* strain.

The biologically active compound is any compound having a biological activity useful in human or animal food, as dietary supplements, for immunomodulation or as a medicament. It can be any organic or inorganic compound, preferably an organic compound, such as a protein, a lipid or a polysaccharide. The molecular mass in weight (MW) of this compound (*e.g*., an antibacterial agent) is advantageously comprised between 100 and 10 000 g/mole.

Advantageously, the concentration of the biologically active compound in the culture medium is 0.5 to 5 mg/ml, preferably 0.5 to 3 mg/ml and more preferably 1 to 2 mg/ml.

In a preferred embodiment, the biologically active compound for use in human or animal is a medicament, preferably an antibacterial agent.

This antibacterial agent can belong to a family selected from the group consisting of macrolides, aminoglycosides, nitrofurans and phenicols.

By way of example, it is clarithromycin, erythromycin, thiamphenicol, amikacine or nitrofurantoin.

As used herein, heat stable means that the biologically active compound is stable at temperature up to at least 55°C.

Advantageously, the biologically active compound is stable at temperature up to 55°C, preferably up to 65°C.

Advantageously, the pH of the hormetin rich medium is comprised between 5 and 8, preferably between 6 and 7.5.

As used herein, a hormetin rich medium as defined in the claims refers to a yeast culture medium suitable for cultivating a yeast cell under aerobic conditions and containing a hormetin.

Yeast culture media suitable for cultivating a yeast cell under aerobic conditions are well-known in the art.

Advantageously, a yeast culture media suitable for cultivating a yeast cell under aerobic conditions comprises oligo- and poly-saccharides, lipids (such as phospholipids), a protein hydrolysate, a buffer, vitamins, minerals and microelements.

The hormetin rich medium can be a liquid or solid medium, preferably a liquid medium (broth).

In a preferred embodiment, the hormetin rich medium comprises organic nitrogen and a hormetin.

In another preferred embodiment, the hormetin rich medium comprises cereal or protein alkaline hydrolysate and/or potato starch (*e.g*., mashed red or sweet potatoes) and a hormetin.

The potato tuber may contain micronutrients that improve the incorporation of the biologically active compound into the yeast cell, such as amylopectine, amylose, and a great variety of protids and glycoproteins (*e.g*., lipid hydrolases with patatin domain). Adding polysaccharides and colloids, such as starch, increases the resistance of yeast cells to heat.

In a particular embodiment, the cereal alkaline hydrolysate is a hydrolyzed filtrated cereal juice made of wheat, oat, barley, rye, buckwheat, rice, or potato flour.

In another particular embodiment, the cereal alkaline hydrolysate is spelt wheat flour that is alkaline hydrolyzed by autoclaving with potassium hydroxide.

By way of example, the hormetin rich medium comprises 30 to 35% volume of filtrated alkaline hydrolyzed spelt wheat until to obtain a pH of 6.5.

In a particular embodiment, the hormetin rich medium is only red grape and/or berry juice, preferably organic (*i.e.,* from organic farming).

As used herein, the term hormetin refers to a chemical compound that is beneficial to the growth of the yeast cell at a low level (*i.e.,* a low concentration) but harmful (*e.g.,* lethal or inducing an inhibition of the growth) for said yeast at a higher level (Calabrese, 2008; Calabrese *et al.,* 1999). It is therefore a mild stress-inducing compound that increases the homeodynamic space of a yeast cell in terms of an increased defence capacity and a reduced load of damaged macromolecules. Hormetins have a hormesis effect where an iterative exposure to a low dose of a toxic agent (hormetin) results in a beneficial response. Hormetins can slow down both yeast growth and aging. Hormetins are known in the art (Goldberg *et al.,* 2010; Menendez *et al.,* 2013; Rattan *et al.,* 2013). They can be micronutrients such as vitamins and minerals (Hayes, 2010), heavy metals, antibiotics from soil bacteria, ethanol, prooxidants (ozone), pigment micronutrients such as polyphenols (in particular flavonoids and stilbenoids). By way of example of hormetins, resveratrol is a stilbene polyphenol found in diverse plants, such as grapes and other berry fruits, that mediates the beneficial effects of mild environmental stressors on lifespan and health (Markus *et al.,* 2008; Howitz *et al.,* 2008; Markes *et al.,* 2009). Quercetin is a major flavonol found in red wine, grapes and all berries. It was shown that hydrogen peroxide resistance increased in *S. cerevisiae* cells pre-treated with quercetin (Belinha *et al.,* 2007; Dàvalos *et al.,* 2006). Anthocyanins are grape pigments that may be easily absorbed by the yeast cell wall (Caridi, 2007). Jiménez *et al.* (2010) have found that the anthocyanins delphinidin 3-glucoside and petunidin 3-glucoside improve significantly the growth rate of *S. cerevisiae,* indicating that the wine polyphenols delphinidin and petunidin have hormetic activities. Other identified hormetins from plants are fisetin from strawberries, curcumin from Indian spice turmeric, epigallocatechin-3-gallate (a green tea polyphenol) and caffeine.

The hormetin is selected from the group consisting of a polyphenolic compound (*e.g.*, an antifungal polyphenolic compound), an anthocyanin, a flavonoid, a stilbenoid, resveratrol, quercetin, fisetin, curcumin, epigallocatechin-3-gallate, caffeine, a red grape juice, a red berry juice, a pomegranate juice, a blackberry juice, a raspberry juice, a bilberry juice, a blackcurrant juice, a cranberry juice, a beetroot juice and a mixture therefore, preferably red grape juice.

The concentration of the hormetins is generally comprised between 100 nM and 1 mM.

When using a juice as defined above in the method according to the present invention, it is preferably organic and it is advantageously filtered and sterile.

In a particular embodiment, squeezed blackberries or raspberries can be mixed volume/volume with red grape juices of biological grade, considering that their richness in alimentary fibres (pectin, hemicelluloses and cellulose) can partly impede the leakage of the biologically active compound from the enriched yeast membrane structures.

In another particular embodiment, the polyphenolic juices (*e.g*., red grape and/or berry juices) are mixed with pasteurized pulp of raw or sweet potatoes.

Advantageously, the hormetin rich medium comprises red grape and/or berry juice, a pulp of raw or sweet potatoes and a cereal hydrosylate.

For instance, the red grape juice 10ml (33.4%) is mixed with the pulp of raw potatoes 9ml (30%), solution of antibacterial agent (*e.g*., clarithromycin) 2ml (6.6%) and alkaline hydrolyzed cereal juice 9ml (30%), so that the final pH of incorporation broth rises to values around 6.5.

In another preferred embodiment, the hormetin rich medium can be enriched with elemental selenium at a concentration comprised between 10 and 50ppm.

As used herein, the term at least (-)n°C per minute means that n is the lowest absolute value. By way of example, at least 1°C par minute means 1 °C or more *(e.g.,* 1.5, 2, 3, etc.) per minute, and the term at least -2°C par minute means -2°C or less *(e.g.,* -2.5, -3, -3.5, etc.) per minute.

As used herein, the term maintaining the temperature means that the temperature varies not more than ± 1°C, preferably, not more than ± 0.5°C, and more preferably does not vary.

In a preferred embodiment of step a), the temperature is comprised, by order of increasing preference, between 22°C and 27°C, between 23°C and 26°C, between 24°C and 26°C or between 24.5°C and 25.5°C. More preferably, the temperature is 25°C.

In another preferred embodiment of step a), the temperature between 20°C and 28°C is maintained, by order of increasing preference, for 30 to 120 minutes, for 30 to 110 minutes, for 30 to 100, for 30 to 90 minutes, for 40 to 80 minutes, for 50 to 70 minutes, for 55 to 65 minutes or for 60 minutes.

In a preferred embodiment of step b), the temperature is comprised, by order of increasing preference, between 39.5°C and 42.5°C, between 40°C and 42°C or between 40.5°C and 41.5°C. More preferably, the temperature reached in step b) is 41 °C.

In another preferred embodiment of step b), the temperature is increased, in its steepest slope, at a rate per minute, comprised between 1 °C and 8°C, preferably between 2°C and 6°C, more preferably between 3°C and 4°C.

In a preferred embodiment of step c), the temperature is maintained, by order of increasing preference, for 70 to 110 minutes, for 80 to 100 minutes, for 85 to 95 minutes or for 90 minutes.

In a preferred embodiment of step d), the temperature is comprised, by order of increasing preference, between 51.5°C and 52.5°C. More preferably, the temperature reached in step d) is 52°C.

In another preferred embodiment of step d), the temperature is increased, in its steepest slope, at a rate per minute, comprised between 2°C and 8°C, preferably between 3°C and 6°C, more preferably between 4°C and 5°C.

In a preferred embodiment of step e0), the temperature is maintained, by order of increasing preference, for 50 to 70 minutes, for 55 to 65 minutes, or for 60 minutes.

The method according to the present invention can further comprise the following steps:
e1) decreasing the temperature of the culture from the temperature in step e0) to a temperature comprised, by order of increasing preference, between 39°C and 43°C, between 40°C and 42°C or between 40.5°C and 42.5°C, and more preferably at 41 °C, at a rate of at least -2°C per minute; and
e2) maintaining the temperature reached in step e1), by order of increasing preference, for 30 to 90 minutes, for 40 to 80 minutes, for 50 to 70 minutes, for 55 to 65 minutes, or for 60 minutes.

In a preferred embodiment of step e1), the temperature is decreased, in its steepest slope, at a rate per minute, comprised between -2°C and -8°C, preferably between -3°C and - 6°C, more preferably between -4°C and -5°C.

The method according to the present invention can further comprise the following steps:
e3) decreasing the temperature of the culture from the temperature in step e2) to a temperature comprised, by order of increasing preference, between 33°C and 37°C, between 34°C and 36°C, or between 34.5°C and 35.5°C, and more preferably at 35°C, at a rate of at least -2°C per minute; and
e4) maintaining the temperature reached in step e3), by order of increasing preference, for 20 to 60 minutes, for 20 to 50 minutes, for 20 to 40 minutes, for 25 to 35 minutes or for 30 minutes.

In a preferred embodiment of step e3), the temperature is decreased, in its steepest slope, at a rate per minute, comprised between -2°C and -8°C, preferably between -3°C and - 6°C, more preferably between -4°C and -5°C.

The method according to the present invention can further comprise the following steps:
e5) decreasing the temperature of the culture from the temperature in step e0), e2) or e4) to a temperature comprised, by order of increasing preference, between 20°C and 28°C, between 21°C and 28°C, between, 22°C and 28°C, between 23°C and 27°C, between 24°C and 26°C, or between 24.5°C and 25.5°C, more preferably at 25°C, at a rate of at least -2°C per minute; and
e6) maintaining the temperature reached in step e5), by order of increasing preference, for 20 to 60 minutes, for 30 to 50 minutes, for 35 to 45 minutes or for 40 minutes.

In a preferred embodiment of step e5), the temperature is decreased, in its steepest slope, at a rate per minute, comprised between -2°C and -8°C, preferably between -3°C and - 6°C, more preferably between -4°C and -5°C.

In another embodiment of the method according to the present invention, the culture is rotated at 5 to 50 rpm, preferably at 10 to 30 rpm, more preferably at 20 rpm.

In another embodiment of the method according to the present invention, the culture is photoinduced by the polychromatic sunlight, a daylight source or a visible violet wavelength (400 to 430nm).

Advantageously, the photoinduction is applied from step b).

The violet wavelength can be obtained through a colored glass slide, or polyester or polycarbonate color filters, as to copy the natural light reflection on the red grape skins.

The method according to the present invention can further comprise, after step e2 as defined above, the following step: enriching the liquid culture medium (broth) with a phosphate buffer, final concentration 5 to 15 mM.

The phosphate buffer can be in powder form (*e.g*., disodium hydrogen and monopotassium dihydrogen orthophosphate). Consequently, this phosphate powder is thoroughly mixed and dissolved in the liquid culture medium.

In another embodiment of the method according to the present invention, the yeast cell is co-cultured with at least one bacterium strain, preferably a probiotic bacterium strain.

Advantageously, the bacterium strain is inoculated in the hormetin rich medium at 50 to 2000 million cells / ml, preferably at 100 to 500 million cells / ml.

Advantageously, the bacterium strain is a *Lactobacillus* strain, preferably a probiotic *Lactobacillus* strain.

In a particular embodiment, the *Lactobacillus* strain is a *Lactobacillus kefiri* strain. By way of example, it is the strain *Lactobacillus kefiri* BCCM-LMG accession number: 9480 (ATCC 35411).

In another particular embodiment, a *Kluyveromyces* strain and a *Lactobacillus* strain are co-cultured, preferably a *K. marxianus* strain and a *L. kefiri* strain are co-cultured.

Methods for isolating yeast cells cell from a culture medium are well known to the person skilled in the art. By way of example, one can proceed with a centrifugation at a speed of 500 rpm to 2000 rpm for 10 to 30 minutes or with a filtration and then, optionally, proceed with a desiccation of the yeast pellet under gentle atmospheric air stream.

Advantageously, the isolating step takes place immediately after steps e0, e2, e4 or e6 as defined above, at room temperature, preferably between 25°C to 28°C.

The yeast cells can then be mixed with dry cereal flour to obtain, upon rehydration, a live concentrate of biologically active compound biovehicle.

In addition to the preceding features, the invention further comprises other features which will emerge from the following description, which refers to examples illustrating the present invention.

### EXAMPLES

### 1. Materials and methods

### Reagents:

Unless otherwise specified, reagents were purchased from Sigma.

Erythromycin and clarithromycin, belonging to the group of macrolide antibiotics, are routinely prescribed in both human and veterinary medicine. Erythromycin powder was purchased from ABC chemicals. Clarithromycine Biclar (Abbott) is a 500mg powder in a bottle. The erythromycin or clarithromycine concentration in the hormetin rich medium (incorporation broth) ranges from 0.5 to 3 mg/ml, preferably 2mg/ml.

Florfenicol is a fluorinated synthetic analogue of thiamphenicol, routinely prescribed in veterinary medicine. Florfenicol 300mg/ml, Nuflor injectable solution, was purchased from MSD Animal Health. Its final concentration in the hormetin rich medium (broth) is 2mg/ml.

Amikacine sulphate 100mg/2ml: Amukin® injectable solution, is an aminoglycoside antibiotic. It was purchased from Bristol-Myers-Squibb. It is used for treating hospital acquired infections with multidrug resistant gram negative bacteria. It is also used in veterinary medicine. Its final concentration in the hormetin rich medium (broth) broth is 1mg/ml.

Nitrofurantoin is a urinary antibacterial, with a broad spectrum antimicrobial activity. Furadantin MC Pharma Logistics is a macrocrystalline nitrofurantoin: 50 capsules of 50 or 100mg. It was dissolved in dimethylsulfoxide (DMSO) at a concentration of 50mg/ml.

Organic Red Grape Juice Pure Pressed was obtained from Vitamont F47150 Monflanquin or from Biotta Bio Grape Juice (Biotta AG CH8274 Taegerwilen).

Autoclaved or pasteurized squeezed red grapes from organic production have been used as well and give similar results.

Biotta Potato Plus Juice (*i.e.* juice from pressed freshly harvested potatoes enhanced with fernel juice from Biotta AG CH-8274 Taegerwilen) was also used to complement organic red grape juice. But autoclaved or pasteurized pulp of raw or sweet potatoes, best after microfiltration at 0.22µm cut-off, suffice for obtaining excellent results.

### Reference strains and media:

*Streptococcus pyogenes* Rosenbach 1884AL, ATCC12344 comes from the BCCM-LMG Bacteria collection, accession number 21599, laboratorium voor microbiologie, UGent, B-9000 Gent. The culture medium is Brain Heart Infusion Broth of typical composition (g/litre) (brain extract, heart extract and peptones 27.5; D+glucose 2.0; sodium chloride 5.0; di-sodium hydrogen phosphate 2.5; agar-agar (not present in the broth) 15.0. Brain Heart Infusion Agar was purchased from Oxoid CM 0375. The medium may be enriched with a sterilized horse serum from defibrinated horse blood for 20 % of global volume. Brain Heart Infusion SM 225 from Oxoid may also be employed for streptococcus culture broth.
*Escherichia coli* (Migula1895) Castellani and Chalmers 1919AL, ATCC10536. The culture medium is Nutrient agar (Oxoid CM3) in g/litre Lab-Lemco beef extract 1; Yeast extract 2; Peptone 5; NaCl 5; Agar 15; pH7.4
*Lactobacillus paracasei* subsp. *paracasei* Collins, Phillips and Zanoni 1989 VP, type strain of *Lactobacillus casei* subsp. *pseudoplantarum,* came from the BCCM-LMG Bacteria collection, accession number 9192, ATCC 25598, depositor NCIMB. *Lactobacillus kefiri* Kandler and Kunath 1983VL, BCCM-LMG Bacteria collection, accession number 9480, ATCC 35411. A modified Man, Rogosa, Sharpe medium has been used for the *Lactobacillus kefiri* and for *Lactobacillus paracasei* culture, as a reference strain for antibiotic activity of the yeast strains: the composition (g/liter) peptone 10.0; meat extract 8.0; yeast extract 4.0; glucose 20.0; orthophosphate 2.0; magnesium sulphate heptahydrate 0.2; manganese sulphate tetrahydrate 0.05; ferrous sulphate 0.01; agar 10.0 The pH is adjusted to 6.2 with acetic acid.
Washing media are Tris Buffered Saline (TBS), pH to 7.4, or Phosphate Buffered Saline (PBS), pH to 7.4.
Yeast strains: *Saccharomyces cerevisiae* Baker's instant Yeast, *Saccharomyces bayanus* Lalvin QA23, *Saccharomyces cerevisiae* (Desmazières) Meyen, *Saccharomyces bayanus* Saccardo (syn. *Saccharomyces uvarum* Beijerinck), *Saccharomyces boulardii, Kluyveromyces Marxianus* (E.C. Hansen) Van der Walt var. Marxianus). The origin of the yeasts is described above.

The yeast cells (one or several species in a mixture) grow at around 25°C on surface of DYPA culture media (dextrose 20.0gr, yeast extract 5.0gr peptone10.0gr agar 20.0gr water 1 Litre), at least for 48hours, to the point of confluent colonies.

For the experiments, 10 to 15ml of nutritive YPD broth (yeast extract 10.0gr peptone 20.0gr dextrose 20.0gr water 1 Litre) were poured on the DYPA gelose and the yeast colonies were allowed to grow into the liquid medium at ambient temperature and during 20 to 40 minutes so as to obtain a heavy broth insemination.

The yeast cells were then centrifuged at around 1000rpm for 20minutes then restored, after antibiotic (antibacterial agent) drug dispersion, in a mixed hormetin rich medium containing red grape juice or pasteurized squeezed red grapes from biological culture *(i.e.,* red, pink or violet colours of flavonoid mixtures).

For an adequate oxygen supply, the hormetin rich medium containing the *Saccharomyces* strains, enriched with an antibacterial compound, is continuously rotated at room temperature (around 25° C) at a 20 rpm speed (from 10 to 30 rpm) and for 1 to 2 hours. The Laboratory Evaporator, Laborota 4010 from Heidolph Instruments GMBH, Schwabach was used. For industrial applications, an orbital mixing motion of propeller blades, paddle stirrers or partition walls inside a water jacketed glass bioreactor can ensure that the yeasts, juices and atmospheric oxygen were well mixed and that the heat is distributed evenly. Nevertheless, jacketed laboratory reactors for chemical synthesis, with glass propeller stirrers and with 1 to 100 litre capacity (for instance, Radleys, Saffron Walden, Essex CB11 3AZ, UK) are also adequate for industrial applications.

For experimental purposes, the antimicrobial activities of the antibiotic standard solution of known concentration, of the yeasts within their incorporation broth after the methods steps, of the supernatants collected by centrifugation and of the pellets of the centrifuged yeasts, after each washing cycle, were compared, using a Kirby-Bauer Disk Diffusion method.

The selected reference strains were *Streptococcus pyogenes* Rosenbach1884AL (BCCM-LMG 21599) or *Lactobacillus paracasei* subsp. *paracasei* Collins, Phillips and Zanoni 1989 VP (BCCM-LMG 9192).

The streptococci or lactobacilli reference strains populations were counted in a Neubauer cell, and then diluted in PBS so as to obtain 2 million germs in one ml buffer: 0.5 ml of the diluted culture medium were then streaked on the agar plates. Another method for obtaining the suitable density of reference bacteria is the culture medium dilution in PBS so as to obtain with a filter photometer, an optical density of 0.04 at 620nm.

Sterile filter or blotter paper disks were laid down on the gelose, humidified with PBS 3 µl. 6 to 12µl of each above-mentioned fraction were then sucked into the blotting paper disk.

### 2. Similar clarithromycin susceptibility areas of Streptococcus pyogenes on BHI agar and Lactobacilli paracasei on modified MRS agar in the Kirby-Bauer Disk Diffusion method

Numeration of the yeasts: *Saccharomyces* Lalvin grown in YPD broth: 530millions /ml.
Composition of hormetin rich medium (incorporation medium): organic red grape juice 19.8ml - filtrated raw potatoes juice with Selenium standard solution 18µmoles - 9.0 ml clarithromycin Biclar Abbott solution 1.2ml (final concentration 2mg/ml) - final pH: 4.0.
Thermal plates sequence: 25°C 20rpm 60minutes; 41 °C 20rpm 90minutes; 52°C, 20rpm, 60minutes; 41 °C 20rpm 60minutes; 35°C 20rpm 30minutes; 25°C 20rpm 30minutes.

A PBS powder composition (so as to reach a final 15mM concentration) was dissolved in the incorporation medium after the second thermal plate at 41 °C. The pH of this medium, after the dissolution of the powder, does not change significantly (+0.1unit).

5ml of this medium were diluted in 45ml PBS pH 6.5, then centrifuged 20minutes at 1000rpm. The yeast cell pellet (n°1) 100 to 200µl was separated from the supernatant (n°1), then after sampling, the washing procedure was repeated 3 times (3 successive dilutions in 20ml PBS pH 6.5 and 1000rpm centrifugation so as to obtain pellets and supernatants n° 2-3-4, 8µl) to be laid down on the blotter paper disks in the centre of germ streaked agar.

With the *Streptococcus pyogenes* reference strain, the incorporation medium with clarithromycin 2mg/ml produced an inhibition zone diameter of 39.5mm; the watery clarithromycin standard (2mg/ml) exerted a clearance area diameter of 37mm, the inhibition zone diameter of pellet n°3 was 25mm, of pellet n°4, 21mm.

The incorporation ratio (R) of pellet n°4 was 21/39.5=0.53.

With the *Lactobacillus paracasei* reference strain the incorporation medium with clarithromycin 2mg/ml produced an inhibition zone diameter of 40mm; the watery clarithromycin standard (2mg/ml) exerted a clearance area diameter of 38mm, the inhibition zone diameter of pellet n°3 was 22.5mm, of pellet n°4, 20.5mm.

The incorporation ratio (R) of pellet n°4 was 20.5/40=0.51.

The supernatants did not produce any inhibition of reference strains.

### 3. Similar clarithromycin susceptibility areas of Streptococcus pyogenes on BHI agar whether the filtrated raw potatoes juice contains or not elemental selenium 2mM

Numeration of the yeasts: *Saccharomyces* Lalvin grown in YPD broth: 530millions /ml.
Composition of incorporation medium: organic red grape juice 19.8ml - filtrated raw potatoes juice with or without Selenium standard solution 18µmoles - 9.0 ml clarithromycin Biclar Abbott solution 1.2ml (final concentration 2mg/ml) - final pH: 4.0.
Thermal plates sequence: 25°C 20rpm 60minutes; 41°C 20rpm 90minutes; 52°C, 20rpm, 60minutes; 41°C 20rpm 60minutes; 35°C 20rpm 30minutes; 25°C 20rpm 30minutes. A PBS powder composition (so as to reach a final 15mM concentration) was dissolved in the incorporation medium after the second thermal plate at 41°C. The pH of these media, after the dissolution of the powder, does not change significantly (+0.1 unlit).

5ml of these media were diluted in 45ml PBS pH 6.5, then centrifuged 20minutes at 1000rpm The yeast cell pellets (n°1) 100 to 200µl were separated from the supernatant (n°1) then after sampling, the washing procedure was repeated 3 times (3 successive dilutions in 20ml PBS pH 6.5 and 1000rpm centrifugation so as to obtain pellets and supernatants n° 2-3-4, 8µl) to be laid down on the blotter paper disks in the centre of germ streaked agar.

With the *Streptococcus pyogenes* reference strain, the incorporation medium with clarithromycin 2mg/ml and elemental selenium produced an inhibition zone diameter of 39.5mm; the watery clarithromycin standard (2mg/ml) without elemental selenium exerted a clearance area diameter of 37mm, the inhibition zone diameter of pellet n°3 was 25mm, of pellet n°4, 21mm. The incorporation ratio (R) of pellet n°4 was 21/39.5=0.53.

With the *Streptococcus pyogenes* reference strain, the incorporation medium with clarithromycin 2mg/ml but without elemental selenium produced an inhibition zone diameter of 38mm; the inhibition zone diameter of pellet n°3 was 28mm, of pellet n°4, 21mm.

The incorporation ratio (R) of pellet n°4 was 21/38=0.55.

The supernatant n°4 did not produce any inhibition of the reference streptococcus strain.

### 4. Equivalence between red grape and blackberry juice hormetins for providing the vitality of heat stressed Saccharomyces boulardii

Numeration of the yeasts: *Saccharomyces boulardii* grown in YPD broth: 550millions /ml.
Composition of incorporation media: Vitamont organic red grape juice or autoclaved squeezed blackberry juice 19.8ml- Biotta Potato Plus Juice 9ml -Watery solution of clarithromycin Biclar 1.2ml (final concentration 2mg/ml) - final pH 4.0.
Thermal plates sequence: 25°C 20rpm 60minutes; 41 °C 20rpm 90minutes; 52°C, 20rpm, 60minutes; 41°C 20rpm 60minutes; 35°C 20rpm 30minutes; 25°C 20rpm 30minutes.

Violet photoinduction (400-430nm) was applied from the first thermal plate at 41°C.

A PBS powder pH6.5 composition (so as to reach a final 15mM concentration) was dissolved in the incorporation medium after the second thermal plate at 41 °C.

The intact *Saccharomyces boulardii* cells were counted in a Neubauer cell and a wet mount was examined by light microscopy directly from the culture samples.

With a blackberry-potato juice composition, after the thermal plate 25°C 60minutes, 644million cells/ml; after the thermal plate 41 °C 90minutes, 652million cells/ml.

After the thermal plate 52°C 60minutes, 650millon cells/ml; after the thermal plate 41°C 60minutes, 648millon cells/ml; after the thermal plates 35°C 20rpm 30minutes; 25°C 20rpm 30minutes, 626 million cells/ml.

From the first thermal plate 41 °C, numerous 2 to 4 spored asci and free ascospores.

An outstanding *Saccharomyces boulardii* cell growth arrest was observed during the 330 minutes thermal plates applied on the blackberry-potato juice composition.

With a red grape-potato juice composition, after the thermal plate 25°C 60minutes, 582million cells/ml; after the thermal plate 41 °C 90minutes, 580million cells/ml.

After the thermal plate 52°C 60minutes, 612millon cells/ml; after the thermal plate 41°C 60minutes, 544millon cells/ml; asci less frequent and more cell budding. After the thermal plates 35°C 20rpm 30minutes; 25°C 20rpm 30minutes, 638 million cells/ml, numerous large asci but persistent cell budding. The red grape juice hormetins combined with the heat shock showed a similar power balance on the yeast cell chronological life span.

Very similar inhibition zones diameters and incorporation indices (R around 0.4).

### 5. The Streptococcus pyogenes/Lactobacillus paracasei susceptibility areas depend tightly on the plates sequence and upper plate temperature

Numeration of the yeasts: *Saccharomyces* Lalvin grown in YPD broth: 530millions /ml.

The unique culture medium during the thermal plates was YPD broth, pH 6.5.

It was substituted to the preceding thermal plates sequence 25-41-52-41-35-25°C a simplified thermal plates sequence with a mild heat stress at 37°C: 25°C 20rpm 60minutes, 37°C 20rpm 90minutes; a PBS powder pH6.5 composition (so as to reach a final 15mM concentration) was dissolved in the incorporation medium after the thermal plate at 37°C; then 25°C 20rpm 30minutes. The yeast cell pellets were washed 4 times as described in the preceding experiment.

With the *Saccharomyces pyogenes* reference strain, the incorporation medium with clarithromycin 2mg/ml produced an inhibition zone diameter of 38mm; the watery clarithromycin standard (2mg/ml) exerted a clearance area diameter of 38mm; the inhibition zone diameter of pellet n°3 was 0mm, of pellet n°4, 0mm. The incorporation ratio (R) = 0.0

Though, it was observed a regular growth of the *Saccharomyces* Lalvin from 618 to768 million budding cells/ml.

In another experiment, it was substituted to the thermal plates sequence 25-41-52-41-35-25°C an unbalanced thermal plates sequence with a lethal heat stress at 55°C (25°C 20rpm 90minutes; 34°C 20rpm 90minutes; 55°C, 20rpm, 90minutes; 34°C 20rpm 30minutes; 25°C 20rpm 30minutes) without addition of PBS powder to the YPD broth, after the second thermal plate at 34°C. The yeast cell pellets were washed 4 times as described in the preceding experiment.

Numeration of *Saccharomyces* Lalvin grown in YPD induction broth: 498millions /ml.

The unique culture medium during the thermal plates was YPD broth, pH 6.5.
Numeration in Neubauer cell of *Saccharomyces cerevisiae* Lalvin after the first thermal plate of 25°C 90minutes: 640 millions/ml.
Numeration after the sequence 25-34-55°C: 628 millions/ml.
Numeration after the sequence 25-34-55-34-25°C: 478 millions/ml: numerous cell necrosis or vacuolation pictures.

Colony forming efficiency (CFE) is one of the parameters typically used to define growth properties of yeast cells in cultures. The growing colonies number (whose diameter on DYPA agar is equal or larger than 1mm) multiplied by the dilution factor reflects the viable population fraction of the yeasts numerated in Neubauer cell.
CFE of *Saccharomyces cerevisiae* Lalvin after the first thermal plate of 25°C 90minutes in YPD broth: 100%.
CFE of *Saccharomyces cerevisiae* Lalvin after the sequence 25-34-55°C: 0%.
CFE of *Saccharomyces cerevisiae* Lalvin after the sequence 25-34-55-34-25°C: 0%.
Disk Diffusion method: with the *Lactobacillus paracasei* reference strain, the YPD incorporation medium with clarithromycin 2mg/ml produced an inhibition zone diameter of 41mm; the watery clarithromycin standard (2mg/ml) exerted a clearance area diameter of 40.5mm.

After the sequence of 25-34°C, and the washings of the *Saccharomyces* pellets the inhibition zone diameter of pellet n°3 was 10.5mm, of pellet n°4, 11mm. The incorporation ratio (R) of pellet n°4 was 11/41=0.27.

After the sequence of 25-34-55°C, and the washings of the *Saccharomyces* pellets, the inhibition zone diameter of pellet n°3 was 15mm, of pellet n°4, 10.5mm. The incorporation ratio (R) of pellet n°4 was 10.5/41=0.26.

After the sequence of 25-34-55-34-25°C, and the washings of the *Saccharomyces* pellets, the inhibition zone diameter of pellet n°3 was 16mm, of pellet n°4, 10mm. The incorporation ratio (R) of pellet n°4 was 10/41=0.24.

The supernatants above these pellets did not produce any inhibition of reference strains.

These incorporation ratios did not increase throughout the thermal plates and may represent passive adsorption on cell membranes.

### 6. Saccharomyces bayanus Lalvin enhanced erythromycin incorporation capacity around pH neutrality

Numeration of the yeast cells grown in YPD broth: broth 30ml: 572million cells/ml.
Composition of incorporation medium: Vitamont organic red grape juice 19.5ml (65%)- Alkaline hydrolyzed spelt wheat flour 8.4ml (28%)- autoclaved parsley juice 2,1ml (7%) - final pH 7.0.

Erythromycin powder (ABC chemicals) was dissolved in the incorporation medium so as to get a final concentration of 1mg/ml.
Thermal plates sequence: 25°C 20rpm 60minutes; 41°C 20rpm 90minutes; 52°C, 20rpm, 60minutes; 41°C 20rpm 60minutes; 35°C 20rpm 30minutes; 25°C 20rpm 30minutes. Violet photoinduction was applied from the first thermal plate at 41 °C.
Reference strain: *Streptococcus pyogenes* on BHI agar enriched with defibrinated horse blood.
Erythromycin loaded yeast incorporation medium after the last thermal plate: 45mm.
Erythromycin standard (2mg/ml): 48mm inhibition zone.
Yeast pellet n°3: 30mm inhibition zone, yeast pellet n°4: 30mm inhibition zone - R=0.66.

The supernatants n° 3 and 4 showed no inhibition area.

### 7. Progressive clarithromycin incorporation into Saccharomyces bayanus Lalvin throughout the thermal plates and at pH 6.5

Numeration of the *Saccharomyces bayanus* Lalvin in YPD broth: 518 millions/ml.
Composition of incorporation medium: organic red grape juice 14.8ml - Biotta Potato Plus Juice 9ml - Alkaline hydrolyzed spelt wheat flour 5ml - Watery solution of clarithromycin Biclar Abbott 1.2ml (final concentration 2mg/ml) - final pH 6.5.
Reference strain: *Lactobacillus paracasei.*
Thermal plates sequence: 25°C 20rpm 60minutes; 41°C 20rpm 90minutes; 52°C, 20rpm, 60minutes; 41°C 20rpm 60minutes; 35°C 20rpm 30minutes; 25°C 20rpm 30minutes.

A PBS powder pH6.5 composition (so as to reach a final 15mM concentration) was dissolved in the incorporation medium after the second thermal plate at 41 °C.
Reference strain: *Lactobacillus paracasei.*
Clarithromycin loaded yeast incorporation medium before the thermal plates: 39mm.
Clarithromycin loaded yeast incorporation medium after the last thermal plate: 39.5mm.
Clarithromycin standard (2mg/ml): 38.5mm.

Yeast aliquots were washed 3 times, after each thermal plate, then 8µl pellet were laid down on reference strain streaked agar.
Yeast pellet n°3 after incubation 1h, 25°C: 11mm inhibition zone - R=0.28.
Yeast pellet n°3 after incubation 1h30, 41 °C: 15.5mm inhibition zone - R=0.39.
Yeast pellet n°3 after incubation 1h, 52°C: 23mm inhibition zone - R=0.58.
Yeast pellet n°3 after incubation sequence 25-41-52-41°C: 23mm inhibition zone - R=0.58.
Yeast pellet n°3 after incubation sequence 25-41-52-41-35-25°C: 23.5mm inhibition zone- R=0.60. The supernatants produced no clearing area (0mm).
Numeration (after dilution in Neubauer cell) of *Saccharomyces bayanus* Lalvin, after the sequence 25-41-52°C: 500millions/ml.
Numeration (after dilution in Neubauer cell) of *Saccharomyces bayanus* Lalvin, after the sequence 25-41-52-41°C: 526millions/ml: nowhere, any significant cell necrosis picture but outstanding prolonged chronological life span with arrest of cell cycle, from in YPD broth yeast transfer to the end of the thermal plates sequence in incorporation medium.

### 8. Optimal development of thermotolerance around 41°C at pH 6.5

This experiment compares the "R" index after 60 or 120 minutes mild heat shock preconditioning at 41 °C with a weaker incorporation index after a thermotolerance induction of 120minutes.

Numeration of the yeasts *Saccharomyces* Lalvin grown in YPD broth: 504millions /ml.

No cell agglutination after dilution in Neubauer cell.
Composition of incorporation medium: Vitamont organic red grape juice 14.8ml - Biotta Potato Plus Juice 9ml - Alkaline hydrolyzed spelt wheat flour 5ml - Watery solution of clarithromycin Biclar Abbott 1.2ml (final concentration 2mg/ml) - final pH 6.5.
Thermal plates sequence: 25°C 20rpm 60minutes; 41 °C 20rpm 60 or 120minutes; 52°C, 20rpm, 60minutes; 41°C 20rpm 60minutes; 35°C 20rpm 30minutes; 25°C 20rpm 30minutes.

Violet photoinduction was applied from the first thermal plate at 41 °C.

A PBS powder pH6.5 composition (so as to reach a final 15mM concentration) was dissolved in the incorporation medium after the second thermal plate at 41 °C.

### Reference strain: Lactobacillus paracasei.

Numeration after the sequence 60minutes 41 °C 60minutes 52°C: 494 millions/ml.
Numeration after the sequence 120minutes 41°C 60minutes 52°C: 482 millions/ml with a tendency to cell agglutination.
Numeration after the sequence 60minutes 41°C 60minutes 52°C 60minutes 41°C: 530 millions/ml.
Numeration after the sequence 120minutes 41 °C 60minutes 52°C 60minutes 41 °C: 418 millions/ml.
Numeration after the sequence 60minutes 41 °C 60minutes 52°C 60minutes 41 °C 30minutes 35°C then 25°C: 502 millions/ml.
Numeration after the sequence 120minutes 41°C 60minutes 52°C 60minutes 41°C 30minutes 35°C then 25°C: 482 millions/ml.
Clarithromycin loaded yeast incorporation medium after the last thermal plate: 41mm after 60minutes at 41 °C and 40.5 mm after 120minutes at 41 °C.
Yeast pellet n°3 after the whole thermal plates sequence (with 60minutes at 41 °C): 26.5mm inhibition zone, yeast pellet n°4: 25mm inhibition zone - R=0.61.
Yeast pellet n°3 after the whole thermal plates sequence (with 120minutes at 41 °C): 25mm inhibition zone, yeast pellet n°4: 22.5mm inhibition zone - R=0.55.

### 9. The heat shock temperature range at pH 6.5 is wide with an optimum around 52°C

Numeration of the yeasts: *Saccharomyces* Lalvin grown in YPD broth: 418millions /ml.

No cell agglutination after dilution in Neubauer cell.
Composition of incorporation medium: Vitamont organic red grape juice 14.8ml - Biotta Potato Plus Juice 9ml - Alkaline hydrolyzed spelt wheat flour 5ml - Watery solution of clarithromycin Biclar Abbott 1.2ml (final concentration 2mg/ml) - final pH 6.5.
Thermal plates sequence: 25°C 20rpm 60minutes; 41°C 20rpm 90minutes; 45 or 47 or 49 or 52°C, 20rpm, 60minutes; 41°C 20rpm 60minutes; 35°C 20rpm 30minutes; 25°C 20rpm 30minutes.

Violet photoinduction was applied from the first thermal plate at 41 °C.

### Reference strain: Lactobacillus paracasei.

A PBS powder pH6.5 composition (so as to reach a final 15mM concentration) was dissolved in the incorporation medium after the second thermal plate at 41 °C.

For the sequence 25-41-45-41-35-25°C pellet n°3 inhibition zone was 18.5mm; Pellet n°4 was 17mm (R=0.42).

For the sequence 25-41-47-41-35-25°C pellet n°3 inhibition zone was 21.5mm; Pellet n°4 was 20mm (R=0.51).

For the sequence 25-41-49-41-35-25°C pellet n°3 inhibition zone was 21mm; Pellet n°4 was 20.5mm (R=0.51).

For the sequence 25-41-52-41-35-25°C pellet n°3 inhibition zone was 24mm; Pellet n°4 was 22.5mm (R=0.58).

### 10. Saccharomyces viability parallelism with the numeration fluctuations

Colony forming efficiency (CFE) is one of the parameters typically used to define growth properties of yeast cells in cultures. The growing colonies number (whose diameter on DYPA agar is equal or larger than 1mm) multiplied by the dilution factor reflects the viable population fraction of the yeasts numerated in Neubauer cell.

It is feasible to clarify the parallel status of cell morphology, numeration fluctuations in Neubauer cells and colony plating's on dishes, for assessing the viability of the *Saccharomyces* strains immediately after each plate of the usually lethal heat shock, here the viability of *Saccharomyces bayanus* Lalvin QA23 or *Saccharomyces bayanus* Saccardo.
Composition of incorporation medium: Vitamont organic red grape juice 19.8ml - filtrated raw potatoes juice with Selenium standard solution 18µmoles, 9.0 ml - solution of clarithromycin Biclar 1.2ml (final concentration 2mg/ml) - final pH: 4.0.
Thermal plates sequence: 25°C 20rpm 30minutes; 41°C 20rpm 90minutes; 52°C, 20rpm, 60minutes; 41°C 20rpm 60minutes; 35°C 20rpm 30minutes.

Violet photoinduction was applied from the first thermal plate at 41 °C.

A PBS powder pH6.5 composition (so as to reach a final 15mM concentration) was dissolved in the incorporation medium after the second thermal plate at 41 °C.

A skullcap extract (as 3% of incorporation medium volume) was diluted after the 35°C thermal plate. The yeast incorporation whole medium was diluted from 100 to 2 million times with PBS 15mM pH6.5 (eventually enriched with 1% Saponaria officinalis extract), then 200µl PBS were laid down on DYPA agar so as to allow 50 to 300 actively growing colonies within 48hours.

### Reference strain for Kirby-Bauer disk diffusion method: Lactobacillus paracasei.

Numeration of the yeast cells: *Saccharomyces bayanus* Lalvin grown in YPD broth: 342 million cells/ml.
Numeration after the sequence 30minutes 25°C: 472 million/ml - CFE 100%.
Numeration after the sequence 25-41-52°C: 494 million/ml - CFE 78%.
Numeration after the sequence 25-41-52-41-35°C: 452million/ml - CFE100%.
Numeration of the yeast cells: *Saccharomyces bayanus* Saccardo grown in YPD broth: 616 million cells/ml.
Numeration after the sequence 30minutes 25°C: 694 million/ml - CFE 98%.
Numeration after the sequence 25-41-52°C: 678 million/ml - CFE 43%.
Numeration after the sequence 25-41-52-41-35°C: 580million/ml - CFE 93%.

For both yeast strains, active sporulation microscopic images, but the *Saccharomyces bayanus* Saccardo cells, although more numerous in YPD broth, showed a greater loss in immediate viability after heat shock at 52°C.
Disk Diffusion method: clarithromycin loaded *Saccharomyces bayanus* Lalvin incorporation medium after the last thermal plate: 41.5mm.
Clarithromycin standard (2mg/ml): 42mm inhibition zone.
Yeast pellet n°3 after the whole thermal plates sequence: 18mm inhibition zone, yeast pellet n°4: 16.5mm inhibition zone - R=0.40.
Clarithromycin loaded *Saccharomyces bayanus* Saccardo incorporation medium after the last thermal plate: 40.5mm.
Yeast pellet n°3 after the whole thermal plates sequence 20.5mm inhibition zone, yeast pellet n°4: 20.0mm inhibition zone - R=0.49.

### 11. Influence of the pH of the Saccharomyces Lalvin incorporation medium

The antibiotic incorporation index was generally lower than 0.5 when the medium pH was set between 3.5 and 4.0: in particular with organic red grape or berry juices compositions with or without potato juice. This index was generally higher than 0.5 when the medium pH was set between 6.0 and 7.0: in particular after addition of alkali or alkaline hydrolyzed wheat flour to neutralize the red grape or berry juices organic acids.
For instance, numeration of the *Saccharomyces* Lalvin grown in YPD broth: 394 millions /ml.
Composition of incorporation medium: Vitamont organic red grape juice 19.8ml - filtrated raw potatoes juice with Selenium standard solution 18µmoles, 9.0 ml - solution of clarithromycin Biclar 1.2ml (final concentration 2mg/ml) - final pH: 4.0.

Compared to Vitamont organic red grape juice 14.8ml - Biotta Potato Plus Juice 9ml - Alkaline hydrolyzed spelt wheat flour 5ml - solution of clarithromycin Biclar 1.2ml (final concentration 2mg/ml) - final pH 6.5.

Thermal plates sequence: 25°C 20rpm 60minutes; 41°C 20rpm 90minutes; 52°C, 20rpm, 60minutes; 41°C 20rpm 60minutes; 35°C 20rpm 30minutes; 25°C 20rpm 30minutes.

Violet photoinduction was applied from the first thermal plate at 41 °C.

A PBS powder pH6.5 composition (so as to reach a final 15mM concentration) was dissolved in the incorporation medium after the second thermal plate at 41 °C.

### Reference strain: Lactobacillus paracasei.

Numeration after the sequence 90minutes 41°C 60minutes 52°C at pH 4.0: 388 millions/ml.
Numeration after the sequence 90minutes 41 °C 60minutes 52°C at pH 6.5: 348 millions/ml.
Numeration after the sequence 90minutes 41 °C 60minutes 52°C 60minutes 41°C at pH 4.0: 442 millions/ml.
Numeration after the sequence 90minutes 41°C 60minutes 52°C 60minutes 41°C at pH 6.5: 428 millions/ml.
Clarithromycin loaded yeast incorporation medium after the last thermal plate: 41mm (at both pH).
Yeast pellet n°3 after the whole thermal plates sequence (at pH 4.0): 20mm inhibition zone, yeast pellet n°4: 18.5mm inhibition zone - R=0.45.
Yeast pellet n°3 after the whole thermal plates sequence (at pH 6.5): 25mm inhibition zone, yeast pellet n°4: 25mm inhibition zone - R=0.62.

### 12. Saccharomyces Lalvin reduced clarithromycin incorporation capacity at pH 4.0 and whatever the value of the upper thermal plate

At pH 4.0, the "R" values range most of the time from 0.3 to 0.4, depending on the setting of the upper lethal heat temperature.
Composition of incorporation medium: Vitamont organic red grape juice 19.8ml - Biotta Potato Plus Juice, 9.0 ml - solution of clarithromycin Biclar 1.2ml (final concentration 2mg/ml) - final pH: 4.0.
Thermal plates sequence: 25°C 20rpm 60minutes; 41°C 20rpm 90minutes; 41°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the second thermal plate at 41 °C.

### Reference strain: Lactobacillus paracasei.

Numeration of the yeast cells: *Saccharomyces* Lalvin grown in YPD broth: 392 million cells/ml.
Clarithromycin loaded yeast incorporation medium after the last thermal plate: 39mm.
Clarithromycin standard (2mg/ml): 41mm inhibition zone.
Yeast pellet n°3: 14mm inhibition zone, yeast pellet n°4: 14mm inhibition zone - R=0.36.
Thermal plates sequence: 25°C 20rpm 60minutes; 41°C 20rpm 90minutes; 43°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the thermal plate at 43°C.

### Reference strain: Lactobacillus paracasei.

Numeration of the yeast cells: *Saccharomyces* Lalvin grown in YPD broth: 494 million cells/ml.
Clarithromycin loaded yeast incorporation medium after the last thermal plate: 39.5mm.
Clarithromycin standard (2mg/ml): 41mm inhibition zone.
Yeast pellet n°3: 17.5mm inhibition zone, yeast pellet n°4: 15.5mm inhibition zone - R=0.39.
Thermal plates sequence: 25°C 20rpm 60minutes; 41°C 20rpm 90minutes; 45°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the thermal plate at 45°C.
Numeration of the yeast cells: *Saccharomyces* Lalvin grown in YPD broth: 494 million cells/ml.
Clarithromycin loaded yeast incorporation medium after the last thermal plate: 39mm.
Clarithromycin standard (2mg/ml): 41mm inhibition zone.
Yeast pellet n°3: 19mm inhibition zone, yeast pellet n°4:15.5mm inhibition zone - R=0.40.
Thermal plates sequence: 25°C 20rpm 60minutes; 41°C 20rpm 90minutes; 47°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the thermal plate at 47°C.
Numeration of the yeast cells: *Saccharomyces* Lalvin grown in YPD broth: 504million cells/ml.
Clarithromycin loaded yeast incorporation medium after the last thermal plate: 39mm.
Clarithromycin standard (2mg/ml): 38.5mm inhibition zone.
Yeast pellet n°3: 17.5mm inhibition zone, yeast pellet n°4: 15mm inhibition zone - R=039.
Thermal plates sequence: 25°C 20rpm 60minutes; 41°C 20rpm 90minutes; 49°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the thermal plate at 49°C.
Numeration of the yeast cells: *Saccharomyces* Lalvin grown in YPD broth: 504million cells/ml.
Clarithromycin loaded yeast incorporation medium after the last thermal plate: 41mm.
Clarithromycin standard (2mg/ml): 40mm inhibition zone.
Yeast pellet n°3: 17.5mm inhibition zone, yeast pellet n°4: 15.5mm inhibition zone - R=0.38.
Thermal plates sequence: 25°C 20rpm 60minutes; 41°C 20rpm 90minutes; 51°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the thermal plate at 51 °C.
Numeration of the yeast cells: *Saccharomyces* Lalvin grown in YPD broth: 604 million cells/ml.
Clarithromycin loaded yeast incorporation medium after the last thermal plate: 40mm.
Clarithromycin standard (2mg/ml): 40mm inhibition zone.
Yeast pellet n°3: 17.5mm inhibition zone, yeast pellet n°4: 15.5mm inhibition zone - R=0.39.
Thermal plates sequence: 25°C 20rpm 60minutes; 41°C 20rpm 90minutes; 53°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the thermal plate at 53°C.
Numeration of the yeast cells: *Saccharomyces* Lalvin grown in YPD broth: 604 million cells/ml.
Clarithromycin loaded yeast incorporation medium after the last thermal plate: 41mm.
Clarithromycin standard (2mg/ml): 40mm inhibition zone.
Yeast pellet n°3: 17.5mm inhibition zone, yeast pellet n°4: 14.5mm inhibition zone - R=0.35.
Thermal plates sequence: 25°C 20rpm 60minutes; 41°C 20rpm 90minutes; 54°C, 20rpm, 60minutes; 35°C 20rpm 30minutes; 25°C 20rpm 30minutes.

A PBS powder pH6.5 composition (so as to reach a final 15mM concentration) was dissolved in the incorporation medium after the thermal plate at 54°C.
Numeration of the yeast cells: *Saccharomyces* Lalvin grown in YPD broth: 612 million cells/ml.
Clarithromycin loaded yeast incorporation medium after the last thermal plate: 39mm.
Clarithromycin standard (2mg/ml): 40mm inhibition zone.
Yeast pellet n°3: 18.5mm inhibition zone, yeast pellet n°4: 14.5mm inhibition zone - R=0.37.

### 13. Saccharomyces Lalvin better cell survival at 52°C after sufficient induction of the heat shock response pathway

This experiment aims at delimiting a precise mild heat shock modulation that results in increased stress tolerance and extension of lifespan in the general model of alimentary yeasts.
Composition of incorporation medium: Vitamont organic red grape juice 19.8ml - Biotta Potato Plus Juice, 9.0 ml - solution of clarithromycin Biclar Abbott 1.2ml (final concentration 2mg/ml) - final pH: 4.0.
Thermal plates sequence: 25°C 20rpm 60minutes; 35°C 20rpm 90minutes; 52°C, 20rpm, 60minutes; 41 °C 20rpm 60minutes.
Numeration of the yeast cells: *Saccharomyces bayanus* Lalvin grown in YPD broth: 452 million cells/ml.
Numeration of *Saccharomyces bayanus* Lalvin after the sequence 25-35°C: 626 million cells/ml: heavy cell proliferation after the thermal plate induction at 35°C.
Numeration of *Saccharomyces bayanus* Lalvin after the sequence 25-35-52°C: 544 million cells/ml: numerous necrosis images.
Numeration of Saccharomyces Bayanus Lalvin after the sequence 25-35-52-41°C: 542 million cells/ml.
Clarithromycin loaded yeast incorporation medium after the last thermal plate: 40mm.
Clarithromycin standard (2mg/ml): 42mm inhibition zone.
Yeast pellet n°3: 18.5mm yeast pellet n°4: 15mm after sequence 25-35-52°C - R=0.37.
Yeast pellet n°3: 20mm yeast pellet n°4: 17.5mm after sequence 25-35-52-41°C - R=0.44.
Composition of incorporation medium: Vitamont organic red grape juice 19.8ml - Biotta Potato Plus Juice, 9.0 ml - solution of clarithromycin Biclar Abbott 1.2ml (final concentration 2mg/ml) - final pH: 4.0.
Thermal plates sequence: 25°C 20rpm 60minutes; 39°C 20rpm 90minutes; 52°C, 20rpm, 60minutes; 41 °C 20rpm 60minutes.
Numeration of the yeast cells: *Saccharomyces bayanus* Lalvin grown in YPD broth: 452 million cells/ml.
Numeration of *Saccharomyces bayanus* Lalvin after the sequence 25-39°C: 460 million cells/ml.
Numeration of *Saccharomyces bayanus* Lalvin after the sequence 25-39-52°C: 452 million cells/ml: frequent occurrence of cell necrosis images.
Numeration of *Saccharomyces bayanus* Lalvin after the sequence 25-39-52-41°C: 430 million cells/ml.
Clarithromycin loaded yeast incorporation medium after the last thermal plate: 41mm.
Clarithromycin standard (2mg/ml): 42mm inhibition zone.
Yeast pellet n°3: 21.5mm yeast pellet n°4: 17mm after sequence 25-39-52°C - R=0.41.
Yeast pellet n°3: 22mm yeast pellet n°4: 19.5mm after sequence 25-39-52-41°C - R=0.48.

### 14. Independence of clarithromycin incorporation indices from vitamin contents of the culture medium

In this experiment it was intended to compare the growth conditions and clarithromycin incorporation indices of four different incorporation medium compositions:
a--Vitamont organic red grape juice 19.8ml - Biotta Potato Plus Juice 9ml -Watery solution of clarithromycin Biclar 1.2ml (final concentration 2mg/ml) - final pH 4.0.
b-- Vitamont organic red grape juice 28.3ml - Fagron Jecoris Aselli Oleum type A 0.5ml - Watery solution of clarithromycin Biclar 1.2ml (final concentration 2mg/ml) - final pH 3.5 - Cod-liver oil (Jecoris Aselli Oleum type A from Fagron SAS F93200 Saint Denis) (rich in vitamins A and D: Vitamin A 1260 IU/g and Vitamin D3 81 IU/g).

Thermal plates sequence: 25°C 20rpm 60minutes; 41 °C 20rpm 90minutes; 52°C, 20rpm, 60minutes; 41°C 20rpm 60minutes; 35°C 20rpm 30minutes; 25°C 20rpm 30minutes.

Violet photoinduction was applied from the first thermal plate at 41 °C.

A PBS powder pH6.5 composition (so as to reach a final 15mM concentration) was dissolved in the incorporation medium after the second thermal plate at 41 °C.

The reference strain was *Lactobacillus paracasei.*
Numeration of the yeast cells: *Saccharomyces bayanus* Lalvin grown in YPD broth: 460 million cells/ml.
Numeration (after dilution in Neubauer cell) of *Saccharomyces bayanus* Lalvin, after the sequence 25-41-52°C: either in red grape- potato juice medium: 374 millions/ml or in red grape juice plus cod-liver oil: 310 millions/ml.
Numeration (after dilution in Neubauer cell) of *Saccharomyces bayanus* Lalvin, after the sequence 25-41-52-41°C: either in red grape- potato juice medium: 426 millions/ml either in red grape juice plus cod-liver oil: 394 millions /ml.
Clarithromycin loaded red grape- potato juice medium after the last thermal plate: 41mm.
Clarithromycin standard (2mg/ml): 39.5mm inhibition zone.
Yeast pellet n°3: 23mm inhibition zone, yeast pellet n°4: 20mm inhibition zone - R=0.49.
Clarithromycin loaded red grape juice plus cod-liver oil after the last thermal plate: 40mm.
Yeast pellet n°3: 20.5mm inhibition zone, yeast pellet n°4: 17mm inhibition zone - R=0.43.
   c--Vitamont organic red grape juice 14.8ml - Biotta Potato Plus Juice 9ml - Alkaline hydrolyzed spelt wheat flour 5ml - Watery solution of clarithromycin Biclar 1.2ml (final concentration 2mg/ml) - final pH 6.5.
   d-- Vitamont organic red grape juice 23.3ml - Fagron Jecoris Aselli Oleum type A 0.5ml - Alkaline hydrolyzed spelt wheat flour - 5ml Watery solution of clarithromycin Biclar 1.2ml (final concentration 2mg/ml) - final pH 6.5.
Thermal plates sequence: 25°C 20rpm 60minutes; 41°C 20rpm 90minutes; 52°C, 20rpm, 60minutes; 41°C 20rpm 60minutes; 35°C 20rpm 30minutes; 25°C 20rpm 30minutes.

Violet photoinduction was applied from the first thermal plate at 41 °C.

A PBS powder pH6.5 composition (so as to reach a final 15mM concentration) was dissolved in the incorporation medium after the second thermal plate at 41 °C.

The reference strain was *Lactobacillus paracasei.*
Numeration of the yeast cells: *Saccharomyces bayanus* Lalvin grown in YPD broth: 416 million cells/ml.
Numeration (after dilution in Neubauer cell) of *Saccharomyces bayanus* Lalvin, after the sequence 25-41-52°C: either in red grape- potato juice medium: 474 millions/ml either in red grape juice plus cod-liver oil: 420 millions/ml.
Numeration (after dilution in Neubauer cell) of *Saccharomyces bayanus* Lalvin, after the sequence 25-41-52-41°C: either in red grape- potato juice medium: 416 millions/ml either in red grape juice plus cod-liver oil: 394 millions/ml.
Numeration (after dilution in Neubauer cell) of *Saccharomyces bayanus* Lalvin, after the sequence 25-41-52-41-35-25°C: either in red grape - potato juice medium: 490 millions/ml either in red grape juice plus cod-liver oil: 560 millions/ml.
Clarithromycin loaded red grape- potato juice medium after the last thermal plate: 42mm.
Clarithromycin standard (2mg/ml): 36.5mm inhibition zone.
Yeast pellet n°3: 24mm inhibition zone, yeast pellet n°4: 22mm inhibition zone - R=0.52
Clarithromycin loaded red grape juice plus cod-liver oil after the last thermal plate: 41mm.
Yeast pellet n°3: 24mm inhibition zone, yeast pellet n°4: 22.5mm inhibition zone - R=0.55.

### 15. Other yeast strains clarithromycin incorporation indices

In this experiment it was intend to compare the growth conditions and clarithromycin incorporation indices of two morphologically distinct genera: *Saccharomyces* and *Kluyveromyces* more specifically a *Saccharomyces cerevisiae* commercial species conditioned as vacuum packed Baker's Instant Dry yeast (Algist Bruggeman) and a *Kluyveromyces marxianus* Van der Walt var. Marxianus from a BCCM-MUCL strain, accession number 52258 (the origin is a traditional goat's cheese).
Numeration of the yeasts: *Saccharomyces cerevisiae* grown in YPD broth: 588 millions/ml. *Kluyveromyces Marxianus* proliferated more strongly: 764 millions/ml.
Composition of incorporation medium: organic red grape juice 14.8ml - Biotta Potato Plus Juice 9ml - Alkaline hydrolyzed spelt wheat flour 5ml - Watery solution of clarithromycin Biclar 1.2ml (final concentration 2mg/ml) - final pH 6.5.
Thermal plates sequence: 25°C 20rpm 60minutes; 41°C 20rpm 90minutes; 52°C, 20rpm, 60minutes; 41°C 20rpm 60minutes; 35°C 20rpm 30minutes; 25°C 20rpm 30minutes.

Violet photoinduction was applied from the first thermal plate at 41 °C.

A PBS powder pH6.5 composition (so as to reach a final 15mM concentration) was dissolved in the incorporation medium after the second thermal plate at 41°C.

The reference strain was *Lactobacillus paracasei.*

The four usual washings were operated with PBS pH 6.5.

After the thermal plates sequence 41 °C 90minutes, 52°C 60minutes, the numerations and cell aspects were distinct: 584 million Baker's yeasts (*Saccharomyces cerevisiae*) with many necrotic aspects in parallel with 1212 million undamaged *Kluyveromyces marxianus* with a few tubular forms.

After the thermal plates sequence 41 °C 90minutes-52°C 60minutes-41°C 60minutes, the cell aspects were still distinctive 632million Baker's yeasts with many necrotic aspects in parallel with 1154 million intact *Kluyveromyces Marxianus.*

After the whole thermal plates sequence (25-41-52-41-35-25°C.) one may observe a diminished number of Baker's yeasts: 538million/ml and a raising number of *Kluyveromyces marxianus:* 1280million/ml.

Clarithromycin loaded *Kluyveromyces* incorporation medium after the last thermal plate exerted an inhibition of 40.5mm in parallel with *Saccharomyces* which produced the same inhibition zone: 40mm.
*Kluyveromyces* pellet n°3 after the thermal plate's whole sequence (at pH 6.5): 22.5mm inhibition zone, *Kluyveromyces* pellet n°4: 22mm inhibition zone - R=0.54.
*Saccharomyces* pellet n°3 after the thermal plate's whole sequence (at pH 6.5): 24.5mm inhibition zone, *Saccharomyces* pellet n°4: 23mm inhibition zone - R=0.58.

In spite of the drying process and 10% necrotic cells, these Baker's reconstituted yeast cells prove capable of clarithromycin internalization after combined heat-hormetins shock.

### 16. Ktuyveromyces marxianus amikacine sulfate incorporation index

Numeration of the yeasts: *Kluyveromyces marxianus* in YPD broth: 826millions /ml.
Composition of incorporation medium: Vitamont organic red grape juice 20.4ml - Autoclaved squeezed Potatoes Juice enriched with Selenium standard solution 18µmoles, 9ml - 0.6ml of Amukin, final concentration of 1mg/ml - final pH of 4.0.
Thermal plates sequence: 25°C 20rpm 60minutes; 41°C 20rpm 90minutes; 52°C, 20rpm, 60minutes; 41 °C 20rpm 60minutes, then rapid shift to room temperature and 3 washings of the pellets.

Violet photoinduction was applied from the first thermal plate at 41 °C.

### Reference strain: Escherichia coli.

Amikacine sulfate loaded *Kluyveromyces* incorporation medium after the last thermal plate exerted an inhibition of 23mm.
Amikacine sulfate standard in TBS: inhibition of 26.5mm.
*Kluyveromyces* pellet n°2: 11.5mm inhibition zone, *Kluyveromyces* pellet n°3: 12mm inhibition zone.
Supernatants n° 2 or 3: 0mm - R=0.52.

### 17. Association between Saccharomyces cerevisiae and Saccharomyces bayanus Lalvin for florfenicol incorporation

Composition of incorporation medium: organic red grape juice Sautter Pom'or100ml, dextrose 15g/L, ammonium phosphate dibasic 800mg/L glycerol 30% florfenicol 2mg/ml, sodium bicarbonate qs to obtain a pH of 7.5.

Thermal plates sequence: 25°C 20rpm 60minutes; 41°C 20rpm 90minutes; 52°C, 20rpm, 60minutes; 41 °C 20rpm 30minutes.

### Reference strain: Escherichia coli.

Numeration of the combined yeast cells (*Saccharomyces bayanus* Lalvin and *Saccharomyces cerevisiae*)*:* 388million cells/ml.

The first centrifugation wet yeast pellet (1500rpm 15minutes) had an approximate volume of 250µl (8.5% of global incorporation volume).

2 washings of the pellets were operated with TBS 20ml pH 6.5.

Florfenicol loaded yeast incorporation medium after the last thermal plate (20µl deposit): 40mm inhibition zone.

Florfenicol standard 2mg/ml in dimethylsulfoxide: 39mm inhibition zone.
Yeast pellet after first washing: 26mm inhibition zone, supernatant n°1: 20mm.
Yeast pellet after second washing: 23.5mm inhibition zone, supernatant n°2: 0mm R=0.59 for this *Saccharomyces* mixture.

### 18. Association between Kluyveromyces marxianus and Lactobacillus kefiri for clarithromycin incorporation

Numeration of the yeasts: *Kluyveromyces marxianus* in YPD broth 30ml (48 hours culture) 662million cells /ml. Numeration of the *Lactobacilli kefiri* in modified MRS broth 190million cells/ml.
Composition of incorporation medium: Vitamont organic red grape juice 19.6ml - Autoclaved squeezed sweet potatoes juice enriched with Selenium standard solution 18µmoles, 9ml - Watery solution of clarithromycin Biclar 1.4ml (final pH of 4.0).

The *Kluyveromyces marxianus* culture broth was then centrifuged at 1000rpm for 20 minutes and the pellet was diluted in the incorporation medium (30ml). 5ml of undiluted *Lactobacillus kefiri* culture were added to the incorporation medium.

Thermal plates sequence: 25°C 20rpm 60minutes; 41 °C 20rpm 90minutes; 52°C, 20rpm, 60minutes; 41 °C 20rpm 60minutes, then rapid shift to room temperature. Violet photoinduction was applied from the first thermal plate at 41 °C.

### Reference strain: Streptococcus pyogenes.

5ml global clarithromycin incorporation medium were then diluted in 45ml PBS pH 6.5, thoroughly mixed, then centrifuged 1000rpm for 20minutes and the pellet (around 1ml) was washed twice with the same PBS (around 20ml).

Clarithromycin loaded combined incorporation medium exerted an inhibition of 47mm.
Clarithromycin standard in TBS: inhibition of 47mm.
*Kluyveromyces-Lactobacilli* supernatant n°1 39mm; n°2: 24mm; n°3: 0mm inhibition zone in diameter.
*Kluyveromyces-Lactobacilli* pellet n°1 38mm; n°2: 31mm; n°3: 31mm inhibition zone in diameter - R=0.66.

### 19. Kirby-Bauer disk inhibition zones of dried up Saccharomyces cerevisiae Lalvin

The culture and incorporation media were composed of goji, pomegranate, aloe vera juices, alkaline spirulina hydrolysate (each 10 volumes), glycerol (2 volumes) olive oil; jojoba oil and vanilla extract (each 1 volume). The pH was adjusted around 7.0.

The yeast strain was *Saccharomyces cerevisiae* bayanus Lalvin QA23.

Numeration after 36h culture at room temperature, rotary motion of 20rpm, demonstrated a 2 billion cells density/ml. Nitrofurantoin addition 500mg dissolved in dimethylsulfoxide 10ml, (Furadantin MC Pharma Logistics), so as to obtain a concentration of 10mg/ml in the incorporation medium.

After the incubation at room temperature 2 thermal plates were applied: 90minutes at 41°C and 60minutes at 52°C, then a rapid shift to room temperature.

Important relative volume of the yeast cells after 1000rpm, 30minutes centrifugation: 28%.

The global incorporation medium was diluted 10times with Tris Buffered Saline (TBS). Then 2000µl of diluted incorporation medium were membrane filtrated with a cut off of 2.5µm.

2000µl of TBS diluted global incorporation medium represent theoretically (200µlx28%) 56µl yeast cell volume. The membrane was washed twice with 2ml Tris Buffered Saline.

The reference strain was *Escherichia coli.*

Inhibition zone diameters for 20µl deposits: Standard nitrofurantoin in DMSO, 1mg/ml, 32mm; Global incorporation medium nitrofurantoin 10mg/ml, 37mm.

TSB diluted global incorporation medium, nitrofurantoin 1mg/ml, 29mm.

TSB diluted global incorporation medium filtrated (2.5µm cut off), 24mm.

Washed *Saccharomyces* Lalvin, theoretical 56µl deposit on filter membrane, 38.5mm.

2000µl of *Saccharomyces* Lalvin global incorporation medium, nitrofurantoin 10mg/ml were centrifuged, 1000rpm, 30minutes.The weight of the yeast pellet was 140mg.

This pellet was thoroughly dried with atmospheric air, at room temperature, for 4 days.

The brown colored powder was mixed with an equal weight of rye flour.

The reference strain was *Escherichia coli.*

28mg of the mixture (dried up yeasts plus rye flour) were laid down on a 12mm diameter blotter disk, then humidified with 28µl organic red grape juice (whose pH was adjusted to 8.0 with sodium bicarbonate) and involved an inhibition zone of 38mm after an incubation period of 23hours at 30°C, of 42mm after an incubation period of 47hours at 30°C. Simultaneously a yellow aureole of nitrofurantoin appeared with a 27 then 32mm diameter.

42mg deposit of the same mixture gived identical inhibition zones. There was no sign of resistant *Escherichia coli* growth back.

### REFERENCES

Belinha I. et al., 2007; J Agric Food Chem. 55:2446-51
Bishop J.R.P. et al., 1998 ; J. Microencapsul. 15:761-773
Blanquet S. et al., 2003; Appl Environ Microbiol. 69:2884-92
Calabrese E.J et al., 1999; Risk Anal. 19:261-281
Calabrese E.J., 2008; Environ Toxicol Chem. 27:1451-74
Caridi A., 2007; Int J Food Microbiol. 120:167-72
Ciamponi F. et al., 2012; Appl Microbiol Biotechnol. 95:1445-1456
Dardelle G., 2007; Food Hydrocolloids. 21:953-960
Davalos A. et al., 2006; Int J Food Sci Nutr. 57:391-8
Fonseca G.G. et al., 2008; Appl Microbiol Biotechnol. 79:339-54
Fuller R., 1989; J Appl Bacteriol. 66:365-378
Goldberg A.A. et al., 2010; Aging (Albany NY) 2:461-70
Harms H.K. et al., 1987; N Engl J Med. 316:1306-9
Hayes D.P., 2010; Dose Response 8:10-15
Hickson M., 2011; Therap Adv Gastroenterol. 4:185-197
Howitz K.T. et al., 2008; Cell. 133:387-91
Jiménez A. et al., 2010; FEMS Yeast Res. 10:858-69
Johnston B.C. et al., 2011; Cochrane Database Syst Rev. 11:CD004827
Markes F.Z. et al., 2009; J Biochem Cell Biol. 41:2125-8
Markus M.A. et al. 2008; Clin Interv Aging. 3:331-9
Martins F.S. et al., 2007; J Med Microbiol. 56:352-359
Menendez J.A. et al., 2013; Cell Cycle. 12: 555-578
Nelson G., 2002; Int J Pharm. 242:55-62
Nelson G et al., 2006; ACS Symposium Series 923: Washington DC pp. 268-281; S. Svenson, Ed.
Rattan S.I. et al., 2013; Dose Response. 11:99-108
Zanello G. et al., 2009; Curr Issues Mol Biol. 11:47-58

## Claims

1. A method for incorporating a heat stable biologically active compound for use in human or animal into a live yeast cell cultivated under aerobic conditions, comprising the following steps:
a) cultivating a live yeast cell with a heat stable biologically active compound for use in human or animal, in a hormetin rich medium at a temperature comprised between 20°C and 28°C, wherein the hormetin is selected from the group consisting of a polyphenolic compound, an anthocyanin, a flavonoid, a stilbenoid, resveratrol, quercetin, fisetin, curcumin, epigallocatechin-3-gallate, caffeine, a red grape juice, a red berry juice, a pomegranate juice, a blackberry juice, a raspberry juice, a bilberry juice, a blackcurrant juice, a cranberry juice, a beetroot juice and a mixture therefore,
b) increasing the temperature of the culture from the temperature in step a) to a temperature comprised between 39°C and 43°C at a rate of at least 1°C per minute;
c) maintaining the temperature reached in step b) for 60 to 120 minutes;
d) increasing the temperature of the culture from the temperature in step c) to a temperature comprised between 51°C and 53°C at a rate of at least 2°C per minute;
e0) maintaining the temperature reached in step d) for 40 to 80 minutes; and
f) isolating the live yeast cell.

2. The method according to claim 1, **characterized in that** the hormetin rich medium comprises red grape and/or berry juice, a pulp of raw or sweet potatoes and a cereal hydrosylate.

3. The method according to anyone of claim 1 or claim 2, **characterized in that** the temperature reached in step b) is comprised between 40°C and 42°C and the temperature reached in step d) is comprised between 51°C and 53°C.

4. The method according to anyone of claims 1 to 3, **characterized in that** in step a) said temperature is maintained for 30 to 120 minutes.

5. The method according to anyone of claims 1 to 4, **characterized in that** it further comprises the following steps:
e1) decreasing the temperature of the culture from the temperature in step e0) to a temperature comprised between 39°C and 43°C at a rate of at least -2°C per minute; and
e2) maintaining the temperature reached in step e1) for 20 to 90 minutes.

6. The method according to claim 5, **characterized in that** it further comprises the following steps:
e3) decreasing the temperature of the culture from the temperature in step e2) to a temperature comprised between 33°C and 37°C at a rate of at least -2°C per minute; and
e4) maintaining the temperature reached in step e3) for 20 to 60 minutes.

7. The method according to claim 6, **characterized in that** it further comprises the following steps:
e5) decreasing the temperature of the culture from the temperature in step e0), e2) or e4) to a temperature comprised between 20°C and 28°C at a rate of at least -2°C per minute; and
e6) maintaining the temperature reached in step e5) for 20 to 60 minutes.

8. The method according to anyone of claims 1 to 7, **characterized in that** the culture is rotated at 5 to 50 rpm.

9. The method according to anyone of claims 1 to 8, **characterized in that** the culture is photoinduced by the polychromatic sunlight, a daylight source or a visible violet wavelength.

10. The method according to anyone of claims 1 to 9, **characterized in that** the yeast cell is a food grade strain.

11. The method according to anyone of claims 1 to 10, **characterized in that** the yeast cell belongs the family Saccharomycetaceae.

12. The method according to claim 11, **characterized in that** the yeast cell is a *Saccharomyces* strain or a *Kluyveromyces* strain.

13. The method according to anyone of claims 1 to 12, **characterized in that** the biologically active compound is a medicament.

14. The method according to claim 13, **characterized in that** the biologically active compound is an antibacterial agent.

15. The method according to anyone of claims 1 to 14, **characterized in that** the yeast cell is co-cultured with at least one bacterium strain.

## Patentansprüche

1. Verfahren zum Aufnehmen einer hitzestabilen biologisch aktiven Verbindung zur Verwendung in einem Menschen oder Tier in eine lebende Hefezelle, die unter aeroben Bedingungen kultiviert wurde, welches die folgenden Schritte umfasst:
a) Kultivieren einer lebenden Hefezelle mit einer hitzestabilen biologisch aktiven Verbindung zur Verwendung in Menschen oder Tieren, in einem hormetinreichen Medium bei einer Temperatur, die zwischen 20 °C und 28 °C liegt, wobei das Hormetin aus der Gruppe bestehend aus einer Polyphenolverbindung, einem Anthocyanin, einem Flavonoid, einem Stilbenoid, Resveratrol, Quercetin, Fisetin, Curcuma, Epigallocatechin-3-gallat, Coffein, einem roten Traubensaft, einem roten Beerensaft, einem Granatapfelsaft, einem Brombeersaft, einem Himbeersaft, einem Heidelbeersaft, einem Schwarzjohannisbeerensaft, einem Cranberrysaft, einem Rote-Beete-Saft und einer Mischung derselben ausgewählt wird,
b) Erhöhen der Temperatur der Kultur von der Temperatur in Schritt a) auf eine Temperatur, die zwischen 39 °C und 43 °C liegt, mit einer Rate von mindestens 1 °C pro Minute;
c) Halten der Temperatur, die in Schritt b) erreicht wurde, über 60 bis 120 Minuten;
d) Erhöhen der Temperatur der Kultur von der Temperatur in Schritt c) auf eine Temperatur, die zwischen 51 °C und 53 °C liegt, mit einer Rate von mindestens 2 °C pro Minute;
e0) Halten der Temperatur, die in Schritt d) erreicht wurde, über 40 bis 80 Minuten; und
f) Isolieren der lebenden Hefezelle.

2. Verfahren nach Anspruch 1, **dadurch gekennzeichnet, dass** das hormetinreiche Medium Roten Traufen- und/oder Beerensaft, Brei von rohen oder süßen Kartoffeln und ein Getreidehydrolysat umfasst.

3. Verfahren nach einem Anspruch 1 oder Anspruch 2, **dadurch gekennzeichnet, dass** die Temperatur, die in Schritt b) erreicht wurde, zwischen 40 °C und 42 °C liegt und die Temperatur, die in Schritt d) erreicht wurde, zwischen 51 °C und 53 °C liegt.

4. Verfahren nach einem der Ansprüche 1 bis 3, **dadurch gekennzeichnet, dass** in Schritt a) die Temperatur über 30 bis 120 Minuten gehalten wird.

5. Verfahren nach einem der Ansprüche 1 bis 4, **dadurch gekennzeichnet, dass** es ferner die folgenden Schritte umfasst:
e1) Verringern der Temperatur der Kultur von der Temperatur in Schritt e0) auf eine Temperatur, die zwischen 39 °C und 43 °C liegt, mit einer Rate von mindestens -2 °C pro Minute; und
e2) Halten der Temperatur, die in Schritt e1) erreicht wurde, über 20 bis 90 Minuten.

6. Verfahren nach Anspruch 5, **dadurch gekennzeichnet, dass** es ferner die folgenden Schritte umfasst:
e3) Verringern der Temperatur der Kultur von der Temperatur in Schritt e2) auf eine Temperatur, die zwischen 33 °C und 37 °C liegt, mit einer Rate von mindestens -2 °C pro Minute; und
e4) Halten der Temperatur, die in Schritt e3) erreicht wurde, über 20 bis 60 Minuten.

7. Verfahren nach Anspruch 6, **dadurch gekennzeichnet, dass** es ferner die folgenden Schritte umfasst:
e5) Verringern der Temperatur der Kultur von der Temperatur in Schritt e0), e2) oder e4) auf eine Temperatur, die zwischen 20 °C und 28 °C liegt, mit einer Rate von mindestens -2 °C pro Minute; und
e6) Halten der Temperatur, die in Schritt e5) erreicht wurde, über 20 bis 60 Minuten.

8. Verfahren nach einem der Ansprüche 1 bis 7, dadurch gekerznzeichnet, dass die Kultur mit 5 bis 50 U/min gedreht wird.

9. Verfahren nach einem der Ansprüche 1 bis 8, **dadurch gekennzeichnet, dass** die Kultur durch polychromatisches Licht, eine Tageslichtquelle oder eine sichtbare violette Wellenlänge fotoinduziert wird.

10. Verfahren nach einem der Ansprüche 1 bis 9, **dadurch gekennzeichnet, dass** die Hefezelle ein lebensmittelkonformer Stamm ist.

11. Verfahren nach einem der Ansprüche 1 bis 10, **dadurch gekennzeichnet, dass** die Hefezelle zur Familie Saccharomycetaceae gehört.

12. Verfahren nach Anspruch 11, **dadurch gekennzeichnet, dass** die Hefezelle ein *Saccharomyces*-Stamm oder ein *Kluyveromyces*-Stamm ist.

13. Verfahren nach einem der Ansprüche 1 bis 12, **dadurch gekennzeichnet, dass** die biologisch aktive Verbindung ein Medikament ist.

14. Verfahren nach Anspruch 13, **dadurch gekennzeichnet, dass** die biologisch aktive Verbindung ein antibakterielles Mittel ist.

15. Verfahren nach einem der Ansprüche 1 bis 14, **dadurch gekennzeichnet, dass** die Hefezelle zusammen mit mindestens einem Bakterienstamm kultiviert wird.

## Revendications

1. Procédé d'incorporation d'un composé thermostable biologiquement actif destiné à être utilisé chez un humain ou un animal dans une cellule de levure vivante cultivée en condition aérobie, comprenant les étapes suivantes :
a) la culture d'une cellule de levure vivante avec un composé thermostable biologiquement actif destiné à être utilisé chez un humain ou un animal, dans un milieu riche en hormétine à une température comprise entre 20 °C et 28 °C, dans lequel l'hormétine est choisie dans le groupe constitué d'un composé polyphénolique, d'une anthocyanine, d'un flavonoïde, d'un stilbénoïde, du resvératrol, de la quercétine, de la fisétine, de la curcumine, de l'épigallocatéchine-3-gallate, de la caféine, d'un jus de raisin rouge, d'un jus de baie rouge, d'un jus de grenade, d'un jus de mûre, d'un jus de framboise, d'un jus de myrtille, d'un jus de cassis, d'un jus de canneberge, d'un jus de betterave et d'un mélange de ceux-ci,
b) l'augmentation de la température de la culture à partir de la température dans l'étape a) jusqu'à une température comprise entre 39 °C et 43 °C à une vitesse d'au moins 1 °C par minute ;
c) le maintien de la température atteinte dans l'étape b) pendant 60 à 120 minutes ;
d) l'augmentation de la température de la culture à partir de la température dans l'étape c) jusqu'à une température comprise entre 51 °C et 53 °C à une vitesse d'au moins 2 °C par minute ;
e0) le maintien de la température atteinte dans l'étape d) pendant 40 à 80 minutes ; et
f) l'isolement de la cellule de levure vivante.

2. Procédé selon la revendication 1, **caractérisé en ce que** le milieu riche en hormétine comprend un jus de raisin et/ou de baie rouge, une pulpe de pommes de terre crues ou douces et un hydrolysat de céréale.

3. Procédé selon la revendication 1 ou la revendication 2, **caractérisé en ce que** la température atteinte dans l'étape b) est comprise entre 40 °C et 42 °C et la température atteinte dans l'étape d) est comprise entre 51 °C et 53 °C.

4. Procédé selon l'une quelconque des revendications 1 à 3, **caractérisé en ce que**, dans l'étape a), ladite température est maintenue pendant 30 à 120 minutes.

5. Procédé selon l'une quelconque des revendications 1 à 4, **caractérisé en ce qu'**il comprend en outre les étapes suivantes :
e1) la diminution de la température de la culture à partir de la température dans l'étape e0) jusqu'à une température comprise entre 39 °C et 43 °C à une vitesse d'au moins -2 °C par minute ; et
e2) le maintien de la température atteinte dans l'étape e1) pendant 20 à 90 minutes.

6. Procédé selon la revendication 5, **caractérisé en ce qu'**il comprend en outre les étapes suivantes :
e3) la diminution de la température de la culture à partir de la température dans l'étape e2) jusqu'à une température comprise entre 33 °C et 37 °C à une vitesse d'au moins -2 °C par minute ; et
e4) le maintien de la température atteinte dans l'étape e3) pendant 20 à 60 minutes.

7. Procédé selon la revendication 6, **caractérisé en ce qu'**il comprend en outre les étapes suivantes :
e5) la diminution de la température de la culture à partir de la température dans l'étape e0), e2) ou e4) jusqu'à une température comprise entre 20 °C et 28 °C à une vitesse d'au moins -2 °C par minute ; et
e6) le maintien de la température atteinte dans l'étape e5) pendant 20 à 60 minutes.

8. Procédé selon l'une quelconque des revendications 1 à 7, **caractérisé en ce que** la culture est en rotation à 5 à 50 tours/minute.

9. Procédé selon l'une quelconque des revendications 1 à 8, **caractérisé en ce que** la culture est photoinduite par la lumière polychrornatique du soleil, une source de lumière du jour ou une longueur d'onde violette visible.

10. Procédé selon l'une quelconque des revendications 1 à 9, **caractérisé en ce que** la cellule de levure est une souche de qualité alimentaire.

11. Procédé selon l'une quelconque des revendications 1 à 10, **caractérisé en ce que** la cellule de levure appartient à la famille *Saccharomycetaceae.*

12. Procédé selon la revendication 11, **caractérisé en ce que** la cellule de levure est une souche de *Saccharomyces* ou une souche de Kluyveromyces.

13. Procédé selon l'une quelconque des revendications 1 à 12, **caractérisé en ce que** le composé biologiquement actif est un médicament.

14. Procédé selon la revendication 13, **caractérisé en ce que** le composé biologiquement actif est un agent antibactérien.

15. Procédé selon l'une quelconque des revendications 1 à 14, **caractérisé en ce que** la cellule de levure est co-cultivée avec au moins une souche de bactérie.
